(19) Europäisches Patentamt
European Patent Office
Office européen des brevets

(11) Publication number: **0 076 622**
**A1**

(12) **EUROPEAN PATENT APPLICATION**

(21) Application number: 82305126.3

(22) Date of filing: 29.09.82

(51) Int. Cl.³: C 07 D 513/04
A 61 K 31/425
//(C07D513/04, 277/00, 235/00)

(30) Priority: 07.10.81 GB 8130241
08.06.82 GB 8216624

(43) Date of publication of application: 13.04.83 Bulletin 83/15

(84) Designated Contracting States: BE CH DE FR GB IT LI NL SE

(71) Applicant: BEECHAM GROUP PLC
Beecham House Great West Road
Brentford Middlesex(GB)

(72) Inventor: Webster, Richard Andrew Bentley
69B Cedar Road
Sutton Surrey(GB)

(72) Inventor: Dorgan, Roderick John
Lingworth Scotshill
Outwood Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

(54) **Anthelmintics.**

(57) Compounds of formula (I):

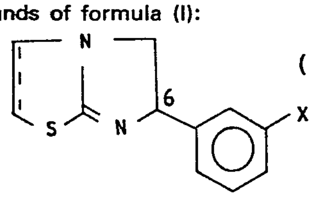

(1)

having the 6-*S*-absolute configuration, or, where appropriate, a salt thereof wherein X is selected from:

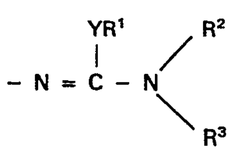

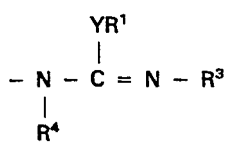

and

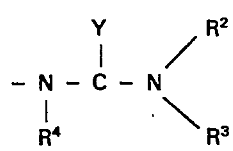

Y is oxygen, or sulphur
$R^1$ is alkyl or aralkyl
$R^2$, $R^3$ and $R^4$ are the same or different and each is selected from hydrogen, alkyl, aryl, substituted aryl, aralkyl, substituted aralkyl, and, in addition $R^2$ and $R^3$ are also selected from heterocyclyl and substituted heterocyclyl, except that when Y is oxygen, $R^2$ and $R^4$ are hydrogen, and the dotted line is an optionally present carbon-carbon bond, have anthelminthic activity.

EP 0 076 622 A1

ANTHELMINTICS

The present invention relates to novel compounds having anthelmintic activity, to processes for their production and to pharmaceutical compositions containing them.

Tetramisole and levamisole are well known anthelmintics of structure:

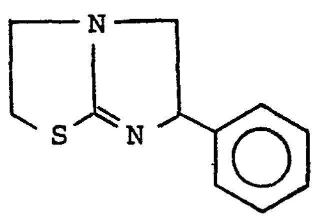

Tetramisole is the dl-form whereas levamisole is the l-form.

It has now been found that certain novel compounds, some of which are derivatives of levamisole, have anthelmintic activity.
In particular these compounds have nematocidal activity, and certain of the compounds also have flukicidal and/or cestodicidal activity.

Accordingly the present invention provides a compound of formula (I):

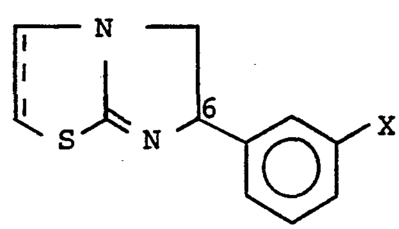

(I)

having the 6-S-absolute configuration, or, where appropriate, a salt thereof wherein X is selected from:

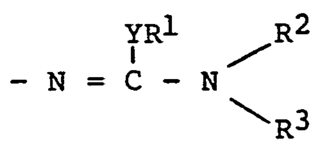

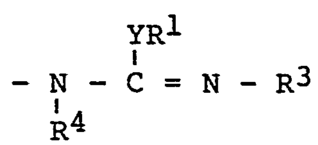

and

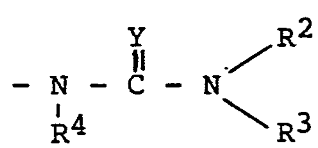

Y is oxygen, or sulphur
$R^1$ is alkyl or aralkyl
$R^2$, $R^3$ and $R^4$ are the same or different and each is selected from hydrogen, alkyl, aryl,

substituted aryl, aralkyl, substituted aralkyl, and, in addition $R^2$ and $R^3$ are also selected from heterocyclyl and substituted heterocyclyl, except that when Y is oxygen, $R^2$ and $R^4$ are hydrogen,

and the dotted line is an optionally present carbon-carbon bond.

Compounds of formula (I) have an asymmetric carbon atom at the 6-position shown above. It is believed that anthelmintic activity is associated with those compounds of formula (I) wherein the 6-position has S-absolute configuration. 6-S-Tetramisole is laevorotatory and, in general, the compounds of formula (I) having 6-S-configuration are also laevorotatory. However, certain compounds of formula (I) may have additional asymmetric carbon atoms and optically pure isomers of such compounds could be dextrorotatory despite having the 6-S-configuration. It is to be understood that the present invention encompasses all compounds of formula (I) having the 6-S-configuration, and such compounds may be optically pure or admixed with stereoisomers, including stereoisomers having the 6-R-absolute configuration.

The isoureas and isothioureas of formula (I), ie those compounds wherein X is:

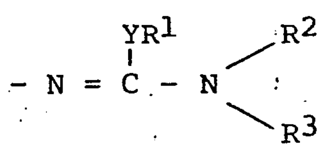

or

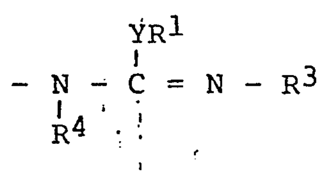

may exist in tautomeric forms, as shown below, when either $R^2$ or $R^4$ is a hydrogen atom:

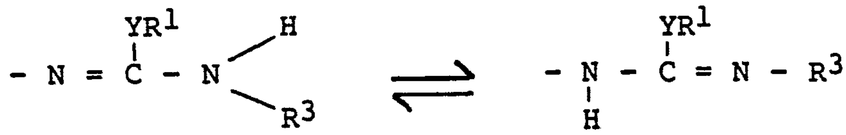

and in such circumstances these two structures may be regarded as equivalent. All such tautomers are encompassed by the invention.

Suitable alkyl groups are $C_{1-10}$ alkyl groups, preferably $C_{1-4}$ alkyl, most preferably methyl or ethyl groups. Alkyl groups having 3 or more carbon atoms may be straight, branched or cyclic.

Suitable aryl groups are monocyclic or fused bicyclic groups, such as phenyl or naphthyl groups, preferably phenyl groups.

Suitable aralkyl groups comprise a monocyclic or fused bicyclic aryl moiety and a $C_{1-10}$ alkylene moiety. Preferably the aryl moiety is phenyl. Preferably the alkylene moiety has from 1 to 4 carbon atoms and most preferably it is methylene.

Suitable heterocyclyl groups include pyridyl and pyrimidyl.

Suitable substituents for the aryl and heterocyclyl groups include halogen, hydroxy, amino, mono- or dialkylamino, nitro, cyano, alkyl, alkoxy, carboxy, alkylcarbonyl, sulphonyl, sulphinyl and trifluoromethyl groups. Conveniently, substituted aryl groups bear up to three substituents. Preferred substituents for the aryl and heterocyclyl groups are fluoro, chloro, nitro, cyano, acetyl and trifluoromethyl groups.

Some of the compounds of formula (I) do not form salts, but where they do suitable salts include the hydrochloride, hydrobromide, hydroiodide, nitrate, sulphate, acetate, citrate, lactate, maleate and pamoate salts.

Preferably salts are pharmaceutically acceptable, however this is not essential as other salts may be useful in the purification of compounds of formula (I) and salts of optically active acids may be useful in the separation of enantiomers of compounds of formula (I).

The isothioureas of formula (I) are particularly advantageous in having flukicidal activity.

In a particular aspect of the present invention there is provided the first medical use of the compounds of formula (I) as hereinbefore defined. Alternatively the invention provides a compound of formula (I) for use in the treatment of the human or animal body, especially for the treatment or prophylaxis of helminthiasis and especially in humans or animals of economic importance such as cattle and sheep, and companion animals such as cats and dogs.

The present invention also provides a process for producing compounds of formula (I) wherein X is a group:

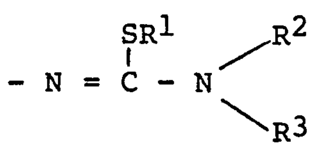

or

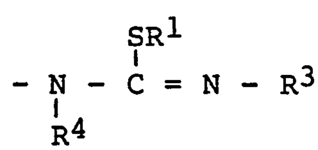

which process comprises reacting a compound of formula (I) or the 6-R-isomer thereof wherein X is a group:

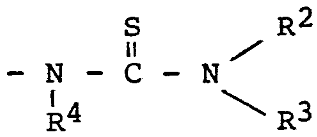

or a salt thereof, wherein at least one of $R^2$, $R^3$ and $R^4$ is hydrogen,
with a compound of formula (II):

$$Z - R^1 \qquad (II)$$

wherein Z is a leaving group such as a halogen atom, and, if necessary, thereafter racemising the product.

Preferably Z is an iodine atom. Conveniently the sodium salt of the sulphur anion of the thiourea of formula (I) is employed.

The reaction may be effected in a conventional manner for converting thioureas to isothioureas. Conveniently the reaction is effected by reacting the compounds of formulae (I) and (II) in an aqueous polar organic solvent in the presence of a strong base.

The present invention further provides a process for producing a compound of formula (I) wherein X is a group:

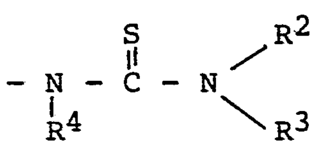

which process comprises either:

(a) reacting a compound of formula (III):

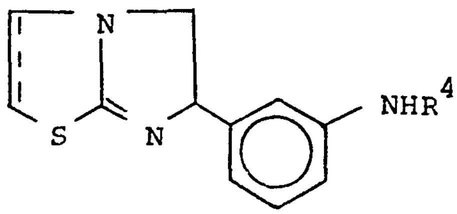

(III)

with a compound of formula (IV):

$$Y = C = N - R^3 \qquad (IV)$$

and, if necessary, thereafter racemising the product,

or (b) reacting the compound of formula (V):

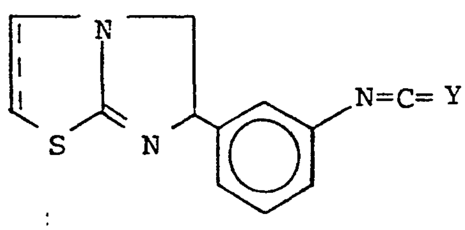

(V)

with a compound of formula (VI):

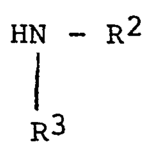

(VI)

and, if necessary, thereafter racemising the product.

The reaction of a compound of formula (III) with a compound of formula (IV) may be effected in conventional manner, for instance in an organic solvent such as acetone, at approximately ambient temperature.

In some circumstances, particularly where the 2,3-double bond is present, the amine of formula (III) reacts with two molecular equivalents of the isothiocyanate (IV) to form a compound thought to be a betaine derivative of formula (VII):

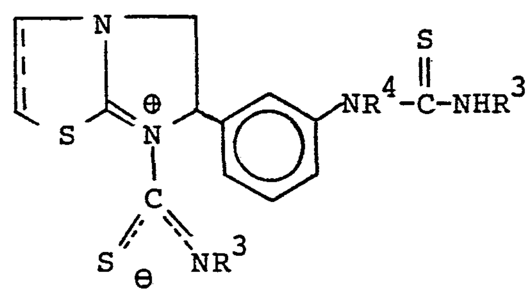

(VII)

Betaine derivatives of this type are formed between tetramisole and several isothiocyanates (J Het Chem, 14, 989 (1977)).

The desired thiourea can be obtained from these betaine thioureas by, for example, treatment with ammonia solution followed by chromatographic purification or fractional crystallisation.

The reaction of a compound of formula (V) with a compound of formula (VI) may be effected in conventional manner, for instance in an organic solvent such as acetone, at an approximately ambient temperature.

The intermediate of formula (V) may be produced by conventional methods, for instance by reacting thiophosgene or an analogue thereof, with a compound of formula (III) wherein $R^4$ is hydrogen, under conventional conditions, such as in aqueous medium in the presence of acid and at elevated temperature, preferably at about 50°C.

The present invention further provides a process for producing a compound of formula (I) or the 6-R-isomer thereof wherein X is

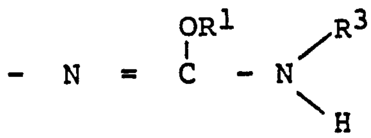

which process comprises converting a compound of formula (I) wherein

X is 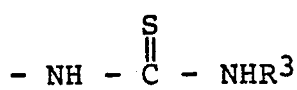 or 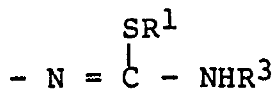

to the required compound of formula (I) and optionally thereafter racemising the product.

The conversion described above may be effected in conventional manner, such as by treating a compound of formula (I) wherein

X is 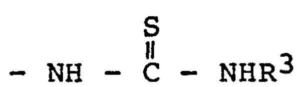

with lead (II) oxide or mercury (II) oxide in the presence of a suitable alcohol, or by treating a compound of formula (I) wherein

X is 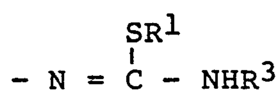

with mercury (II) chloride in the presence of a suitable alcohol.

It will be understood that compounds of formula (I) may be prepared as a mixture of 6-R- and 6-S- isomers by starting from dl-m-aminotetramisole, or a derivative thereof [the amine of formula (III)]. If it is desired to obtain the 6-S-isomer, this may be achieved by using the pure l-m-aminotetramisole. Alternatively, with compounds which form salts, the 6-S-isomer may be obtained from a mixture of 6-R- and 6-S-isomers by resolution in conventional manner. Otherwise the 6-S-isomer may be produced from the l-amine of formula (III), itself prepared by resolution of the corresponding dl-form. Such resolutions may be effected via an optically active salt formed from the racemic compound by reaction with an optically active acid such as d-tartaric acid, (-)-dibenzoyl-L-tartaric acid or d-10 camphorsulphonic acid.

Resolution reactions of this general nature are described in U.K. Patent No. 1 402 689 and U.S. Patent Nos. 3 463 786 and 3 673 205.

Furthermore, the 6-R-isomer of a thiourea of formula (I) may be obtained starting from the 6-R-amine of formula (III), ie d-m-aminotetramisole, then racemised by standard methods. Pure 6-S-isomer may then be produced by resolution of the 6-R-, 6-S- mixture and further 6-S-isomer generated by racemisation of the recovered 6-R-isomer.

Where appropriate, salts of the compounds of the formula (I) may be prepared in entirely conventional manner, for example, by reacting the compounds with the appropriate acid.

The compounds of formula (I) are useful in the treatment or prophylaxis of worm infections in humans and animals, such as cattle and sheep, cats and dogs.

Accordingly, the invention also provides a pharmaceutical or veterinary composition comprising a compound of the formula (I) and a pharmaceutically or veterinarily acceptable carrier thereof.

For oral administration the compositions may conveniently be in the form of solutions or suspensions of the active ingredient. Alternatively, the active ingredient may be mixed in with an animal feedstuff or animal feed supplement.

The compounds of formula (I) may also be administered orally to animals in sustained release dosage forms, such as the rumen devices described in U.K. Patent No. 1 601 923 (German Offenlegungsschrift 28 24 288) or European Patent Applications Numbers A1 0 010 987 and A1 0 021 758.

For parenteral administration the carrier will be any vehicle conveniently used for such administration, for example sterile water, or preferably a formulation in which the carrier comprises from 55 to 85 v/v propylene glycol, 0 to 5% benzylalcohol and sterile water to 100% of the carrier.

Suitably the compositions consist of sufficient material to provide a dose of from 0.1 to 100 mg of active ingredient per kg of animal body weight per dose, more suitably 1 to 50 mg/kg per dose.

The invention also provides a method of treatment or prophylaxis of helminth infections in humans or animals, which comprises the administration to the infected or potentially infected human or animal of an effective non-toxic amount of a compound of formula (I) or a pharmaceutically or veterinarily acceptable salt thereof.

In particular aspects this method includes the treatment or prophylaxis of roundworm infections.

It will be appreciated that it will, in some cases, be advisable to repeat the dosing of the infected or potentially infected human or animal with the compound of formula (I) or a pharmaceutically or veterinarily acceptable salt thereof according to conventional dosage regimes. Examples of such a regime include those used with anthelmintics such as tetramisole and levamisole.

The following Examples illustrate the invention.

EXAMPLE 1

N-Phenyl-N'-[3-(6-{2,3,5,6-tetrahydroimidazo[2,1-b] thiazolyl})phenyl]thiourea

To a solution of 6-(m-aminophenyl)-2,3,5,6-tetrahydroimidazo[2,1-b]thiazole (5g, 22.8 mmol) in acetone (50 ml) at room temperature was added phenylisothiocyanate (3.39g, 25 mmol). The mixture was kept at room temperature overnight, the resulting precipitate collected by filtration, washed with acetone (50 ml) and dried *in vacuo* at 60°C to afford N-Phenyl-N'-[3-(6-{2,3,5,6-tetrahydroimidazo[2,1-b]-tetrahydroimidazo[2,1-b]thiazolyl})phenyl]thiourea (6.75g; 84%).

Found C:61.25; H:5.22; N:15.66; S:17.65
$C_{18}H_{18}N_4S_2$ requires: C:61.01; H:5.08; N:15.82; S:18.08%
m.pt. 133-4°C.

EXAMPLES 2 TO 11

The following compounds were prepared by a method exactly analogous to that of Example 1.

EXAMPLE 2

N-(4-Fluorophenyl)-N'-[3-(6-2,3,5,6-tetrahydroimidazo[2,1-b]thiazolyl)phenyl]thiourea

Found C:58.25; H:4.58; N:14.71; S:17.24
$C_{18}H_{17}FN_4S_2$ requires:C:58.06; H:4.57; N:15.05; S:17.20%
m.pt. 134-135°C

EXAMPLE 3

N-(3-Fluorophenyl)-N'-[3-(6-2,3,5,6-tetrahydroimidazo-[2,1-b]thiazolyl)phenyl]thiourea

Found C:58.20; H:4.78; N:15.16; S:17.43
$C_{18}H_{17}FN_4S_2$ requires:C:58.06; H:4.57; N:15.05; S:17.20%
m.pt. 108-110°C

EXAMPLE 4

N-(4-Nitrophenyl)-N'-[3-(6-2,3,5,6-tetrahydroimidazo-[2,1-b]thiazolyl)phenyl]thiourea

Found C:53.91; H:4.23; N:17.23; S:15.91
$C_{18}H_{17}N_5O_2S_2$ requires:C:54.14; H:4.26; N:17.54; S:16.04
m.pt. 124-6°C

EXAMPLE 5

N-(4-Chlorophenyl)-N'-[3-(6-{2,3,5,6-tetrahydroimidazo-[2,1-b]thiazolyl})phenyl]thiourea

Found C:55.72; H:4.47; N:14.37; S:16.18; Cl:9.51

$C_{18}H_{17}ClN_4S_2$ requires: C:55.60; H:4.38; N:14.41; S:16.47; Cl:9.14%

m.pt. 149-151°C

EXAMPLE 6

N-(4-Methoxyphenyl)-N'-[3-(6-{2,3,5,6-tetrahydro-imidazo[2,1-b]thiazolyl})phenyl]thiourea

Found C:56.95; H:5.74; N:14.20; S:16.48

$C_{19}H_{20}N_4OS_2.H_2O$ requires C:56.71; H:5.47; N:13.93; S:15.92%

m.pt. 153-4°C

EXAMPLE 7

N-(3,4-Dimethylphenyl)-N'-[3-(6-{2,3,5,6-tetrahydro-imidazo[2,1-b]thiazolyl})phenyl]thiourea

Found C:61.46; H:6.24; N:14.25; S:16.41

$C_{20}H_{22}N_4S_2.\frac{1}{2}H_2O$ requires C:61.38; H:5.88; N:14.32; S:16.37%

m.pt. 152-3°C

EXAMPLE 8

N-(3-Methylphenyl)-N'-[3-(6-{2,3,5,6-tetrahydroimidazo-[2,1-b]thiazolyl})phenyl]thiourea

| | | |
|---|---|---|
| Found | | C:59.97; H:5.40; N:14.85; S:17.54 |
| $C_{19}H_{20}N_4S_2$ | requires: | C:61.96; H:5.43; N:15.22; S:17.39% |

m.pt. 98-100°C

EXAMPLE 9

N-(3,4-Dichlorophenyl)-N'-[3-(6-{2,3,5,6-tetrahydro-imidazo[2,1-b]thiazolyl})phenyl]thiourea

| | |
|---|---|
| Found | C:51.03; H:3.72; N:12.58; S:14.78; Cl:16.94 |
| $C_{18}H_{16}Cl_2N_4S_2$ requires: | C:51.06; H:3.78; N:13.25; S:15.31; Cl:16.78% |

m.pt. 163-5°C

EXAMPLE 10

N-(4-Methylphenyl)-N'-[3-(6-{2,3,5,6-tetrahydroimidazo-[2,1-b]thiazolyl})phenyl]thiourea

| | | |
|---|---|---|
| Found | | C:61.54; H:5.39; N:15.50; S:16.60 |
| $C_{19}H_{20}N_4S_2$ | requires: | C:61.96; H:5.43; N:15.22; S:17.39% |

m.pt. 158-160°C

EXAMPLE 11

N-(3-Chlorophenyl)-N'-[3-(6-2,3,5,6-tetrahydroimidazo-[2,1-b]thiazolyl)phenyl]thiourea

| | |
|---|---|
| Found | C:55.69; H:4.65; N:14.17; S:16.38 Cl:9.11 |
| $C_{18}H_{17}Cl_2N_4S_2$ requires: | C:55.60; H:4.38; N:14.41; S:16.47 Cl:9.14% |

m.pt. 165-7°C

0076622

EXAMPLE 12

a) Preparation of 6-(m-isothiocyanatophenyl)-2,3,5,6-tetrahydroimidazo[2,1-b]thiazole

To a solution of 6-(m-aminophenyl)-2,3,5,6-tetrahydroimidazo[2,1-b]thiazole (10.9g) in 2M hydrochloric acid (100 ml) at 50°C was added thiophosgene (4 ml). The temperature was maintained at 50-60°C for 2 h and the solution then cooled to room temperature. The solution was basified with 2M aqueous sodium hydroxide solution and extracted with chloroform (3 x 75 ml). The extracts were dried and evaporated to yield a friable solid (10 g). This material was used without further purification.

b) Preparation of N-(4-methylphenyl)-N'-[3-(6-{2,3,5,6-tetrahydroimidazo[2,1-b]thiazolyl})phenyl]thiourea

To a solution of 6-(m-isothiocyanatophenyl)-2,3,5,6-tetrahydroimidazo[2,1-b]thiazole (4g) in acetone (200ml) was added p-toluidine (2g) and the resulting solution set aside overnight. The precipitate was recovered by filtration, washed with acetone and dried to give N-(4-methylphenyl)-N'-[3-(6-{2,3,5,6-tetrahydroimidazo[2,1-b]thiazolyl})phenyl]thiourea (4.25g), m.pt. 158-9°C.

EXAMPLE 13

S-Methyl-N-phenyl-N'-[3-(6- 2,3,5,6-tetrahydroimidazo-[2,1-*b*]thiazolyl )-phenyl]isothiourea

To *N*-phenyl-N'-[3-(6-2,3,5,6-tetrahydroimidazo-[2,1-*b*]thiazolyl )phenyl]thiourea (5g, 14.1 mmol) suspended in acetone (50ml) was added a solution of sodium hydroxide (565 mg, 14.1 mmol) in water (5ml) and the mixture stirred for one hour at room temperature during which time a clear solution was obtained. The solution was cooled in an ice bath, methyl iodide (0.925 ml, 14.9 mmol) added and the solution stirred for two hours. The acetone was removed in vacuo and the water decanted off from the remaining oil which was taken up in chloroform (50 ml), dried (magnesium sulphate) and concentrated *in vacuo* to afford S-Methyl-N-phenyl-N'-[3-(6- 2,3,5,6-tetrahydro-imidazo[2,1-*b*]-thiazolyl )phenyl]isothiourea (5 g; 96%).

(Found:m/e 368.1132 ($M^+$). Calculated for $C_{19}H_{20}N_4S_2$: *m/e* 368.1130)

To the isothiourea (2 g, 5.43 mmol) in methanol (20 ml) was added isopropanol saturated with hydrogen chloride until an acid solution was obtained. The solvent was removed *in vacuo* and the white solid dried at 60°C *in vacuo* to afford the monohydrochloride salt (2.2 g; 100%), m.pt. 115-8°C (decomposition).

## EXAMPLES 14 TO 25

The following compounds were prepared by a method exactly analogous to that of Example 13.

### EXAMPLE 14

S-Methyl-N-[4-fluorophenyl]-N'-[3-(6-{2,3,5,6-tetrahydroimidazo[2,1-*b*]thiazolyl})phenyl]isothiourea

Found: *m/e* 386.1041 ($M^+$). Calculated for $C_{19}H_{19}FN_4S_2$: *m/e* 386.1035.

### EXAMPLE 15

S-Methyl-N-[3-fluorophenyl]-N'-[3-(6-{2,3,5,6-tetrahydroimidazo[2,1-*b*]thiazolyl})phenyl]isothiourea

Found: *m/e* 386.1072 ($M^+$). Calculated for $C_{19}H_{19}FN_4S_2$: m/e 386.1035.

### EXAMPLE 16

S-Methyl-N-[4-nitrophenyl]-N'-[3-(6-{2,3,5,6-tetrahydroimidazo[2,1-*b*]thiazolyl})phenyl]isothiourea

Found: *m/e* 365.0926 ($[M-HSCH_3]^+$). Calculated for $C_{18}H_{15}N_5O_2S$; *m/e* 365.0946

m.pt. 73-5°C

EXAMPLE 17

S-Methyl-N-[4-chlorophenyl]-N'-[3-(6-{2,3,5,6-tetrahydroimidazo[2,1-*b*]thiazolyl})phenyl]isothiourea

Found: *m/e* 402.0767 ($M^+$). Calculated for $C_{19}H_{19}ClN_4S_2$: *m/e* 402.0738
m.pt. 80-2°C

EXAMPLE 18

S-Methyl-N-[3,4-dichlorophenyl]-N'-[3-(6-{2,3,5,6-tetrahydroimidazo[2,1-*b*]thiazolyl}) phenyl]isothiourea

Found: *m/e* 388.0283 ($[M-HSCH_3]^+$). Calculated for $C_{18}H_{14}Cl_2N_4S$: *m/e* 388.0315

EXAMPLE 19

S-Methyl-N-[4-methylphenyl]-N'-[3-(6-{2,3,5,6-tetrahydroimidazo[2,1-*b*]thiazolyl})phenyl]isothiourea

Found: *m/e* 382.1264 ($M^+$). Calculated for $C_{20}H_{22}N_4S_2$: *m/e* 382.1284.

EXAMPLE 20

S-Methyl-N-[3-chlorophenyl]-N'-[3-(6-{2,3,5,6-tetrahydroimidazo[2,1-b]thiazolyl})phenyl]isothiourea

Found: m/e 402.0748 ($M^+$). Calculated for $C_{19}H_{19}ClN_4S_2$: m/e 402.0738.

m.pt 51-5°C

EXAMPLE 21

S-Methyl-N-[3-methoxyphenyl]-N'-[3-(6-{2,3,5,6-tetrahydroimidazo[2,1-b]thiazolyl})phenyl]isothiourea

Found: m/e 398.1219 ($M^+$). Calculated for $C_{20}H_{22}N_4OS_2$: m/e 389.1234

m.pt. 66-8°C.

EXAMPLE 22

S-Ethyl-N-phenyl-N'-[3-(6-{2,3,5,6-tetrahydroimidazo[2,1-b]thiazolyl})phenyl]isothiourea

Found: m/e 382.1284 ($M^+$). Calculated for $C_{20}H_{22}N_4S_2$: m/e 382.1286.

EXAMPLE 23

S-Octyl-N-phenyl-N'-[3-(6-{2,3,5,6-tetrahydroimidazo-[2,1-*b*]thiazolyl})phenyl]isothiourea

Found m/e 466.2225 ($M^+$). Calculated for $C_{26}H_{34}N_4S_2$: *m/e* 466.2224.

EXAMPLE 24

S-Methyl-N-[4-cyanophenyl]-N'-[3-(6-{2,3,5,6-tetrahydroimidazo[2,1-*b*]thiazolyl})phenyl]isothiourea

Found: m/e 345.1053 ($[M-HSCH_3]^+$). Calculated for $C_{19}H_{15}N_5S$: m/e 345.1047.
m.pt. 61-3°C

EXAMPLE 25

S-Methyl-N-[4-acetylphenyl]-N'-[3-(6-{2,3,5,6-tetrahydroimidazo-[2,1-*b*]thiazolyl})phenyl]isothiourea

Found: *m/e* 362.1190 ($[M-HSCH_3]^+$). Calculated for $C_{20}H_{18}N_4OS$: *m/e* 362.1201.
m.pt. 50-3°C.

EXAMPLES 26 AND 27

The following compounds were prepared by a method exactly analogous to that of Example 1.

EXAMPLE 26

N-(4-Cyanophenyl)-N'-[3-(6-{2,3,5,6-tetrahydroimidazo-[2,1-b]thiazolyl})phenyl]thiourea

Found C:60.05; H:4.58; N:18.38; S:16.56. $C_{19}H_{17}N_5S_2$ requires C:60.16; H:4.49; N:18.47; S;16.89%.
m.pt. 162-4°C

EXAMPLE 27

N-(4-Acetylphenyl)-N'-[3-(6-{2,3,5,6-tetrahydroimidazo-[2,1-b]thiazolyl})phenyl]thiourea

Found C:60.40; H:5.07; N:14.07; S:16.03. $C_{20}H_{26}N_4OS_2$ requires C:60.61; H:5.05; N:14.14; S:16.16%.
m.pt. 133-5°C.

EXAMPLES 28 TO 49

The compounds of Examples 28 to 49 were produced by the method described in Example 1.

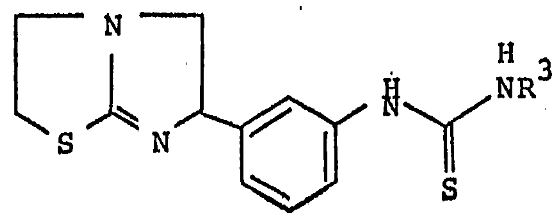

| Example | $R^3$ | Elemental Analysis (%) | | | | | | m.p. (°C) |
|---|---|---|---|---|---|---|---|---|
| | | Found =F<br>Calculated=C | C | H | N | S | Halogen | |
| 28 | 1-naphthyl | F<br>C | 65.10<br>65.35 | 4.90<br>4.95 | 13.85<br>13.86 | 15.63<br>15.84 | | 139-40 |
| 29 | $3-C_6H_4NO_2$ | F<br>C | 54.15<br>54.14 | 4.58<br>4.26 | 16.59<br>17.54 | 16.23<br>16.04 | | 157-60 |
| 30 | $3-C_6H_4Br$ | F<br>C | 50.28<br>49.88 | 4.12<br>3.93 | 12.91<br>12.93 | 14.27<br>14.78 | 18.32<br>18.42 | 172-4 |
| 31 | $4-C_6H_4O\underline{p}NO_2C_6H_4$ | F<br>C | 58.80<br>58.78 | 4.00<br>4.08 | 13.97<br>14.29 | 12.82<br>13.06 | | 175-6 |
| 32 | $2-C_6H_4Cl$ | F<br>C | 55.76<br>55.60 | 4.56<br>4.38 | 14.34<br>14.41 | 16.30<br>16.47 | 8.91<br>9.14 | 118-21 |
| 33 | $4-C_6H_4SO_2NH_2$ | F<br>C | 49.91<br>49.88 | 4.59<br>4.39 | 15.79<br>16.17 | 21.74<br>21.17 | | 148-50 |
| 34 | $4-C_6H_4CO_2C_2H_5$ | F<br>C | 58.50<br>59.15 | 5.28<br>5.16 | 13.22<br>13.15 | 16.14<br>15.02 | | 128-30 |
| 35 | $4-C_6H_4SO_2CH_3$ | F<br>C | 53.73<br>52.78 | 4.83<br>4.63 | 12.56<br>12.96 | 22.59<br>22.22 | | 146-8 |

| Example | $R^3$ | Elemental Analysis (%) | | | | | | m.p. (°C) |
|---|---|---|---|---|---|---|---|---|
| | | Found =F<br>Calculated=C | C | H | N | S | Halogen | |
| 36 | $4-C_6H_4SO_2N$ 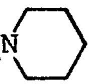 | F<br>C | 55.07<br>55.09 | 5.59<br>5.39 | 13.88<br>13.97 | 19.19<br>19.16 | | 129–31 |
| 37 | $4-C_6H_4SCH_3$ | F<br>C | 56.63<br>57.00 | 5.03<br>5.00 | 13.85<br>14.00 | 23.94<br>24.00 | | 110–5 |
| 38 | $4-C_6H_4SO_2N(CH_3)_2$ | F<br>C | 51.88<br>52.06 | 4.95<br>4.99 | 14.89<br>15.18 | 20.70<br>20.82 | | 120–3 |
| 39 | $4-C_6H_4S\underline{p}NO_2C_6H_4$ | F<br>C | | | | | | 174–6 |
| 40 | $4-C_6H_4CF_3$ | F<br>C | 54.13<br>54.03 | 4.15<br>4.03 | 13.37<br>13.27 | 15.13<br>15.17 | | 159–61 |
| 41 | $4-C_6H_4CO.C_6H_5$ | F<br>C | 65.12<br>65.50 | 4.96<br>4.80 | 12.36<br>12.23 | 13.74<br>13.97 | | 136–40 |
| 42 | $4-C_6H_4SPr$ | F<br>C | 58.68<br>58.88 | 5.46<br>5.61 | 13.16<br>13.08 | 22.34<br>22.43 | | 121–4 |
| 43 | $4-C_6H_4SO.CH_3$ | F<br>C | 54.63<br>54.81 | 4.87<br>4.81 | 12.94<br>13.46 | 23.28<br>23.08 | | 117–20 |
| 44 | $3-C_6H_4CN$ | F<br>C | 60.09<br>60.16 | 4.60<br>4.49 | 18.44<br>18.47 | 16.21<br>16.89 | | 148–50 |

| Example | $R^3$ | Elemental Analysis (%) | | | | | | m.p. (°C) |
|---|---|---|---|---|---|---|---|---|
| | | Found =F<br>Calculated=C | C | H | N | S | Halogen | |
| 45 | $4-C_6H_4SO.Pr$ | F<br>C | 56.16<br>56.76 | 5.25<br>5.41 | 12.67<br>12.61 | 20.63<br>21.62 | | 148–50 |
| 46 | $4-C_6H_4Br$ | F<br>C | 50.27<br>49.88 | 4.07<br>3.93 | 12.84<br>12.93 | 14.20<br>14.78 | | 152–5 |
| 47 | $CH_2C_6H_5$ | F<br>C | 61.76<br>61.96 | 5.64<br>5.43 | 15.26<br>15.22 | 17.25<br>17.39 | | 179–80 |
| 48 | <u>c</u>-$C_6H_{11}$ | F<br>C | | | | | | 95–6 |
| 49 | n-$C_6H_{13}$ | F<br>C | 58.87<br>59.67 | 7.10<br>7.18 | 15.24<br>15.47 | 17.29<br>17.68 | | |

Examples 50 to 78

The compounds of Examples 50 to 78 were produced by the method described in Example 13, except that the compound of Example 54 was produced as described below:

Example 54

O-Methyl-N-phenyl-N'-[3-(6-{2,3,5,6-tetrahydroimidazo-[2,1-*b*]thiazolyl})phenyl]isourea

To a stirred suspension of mercuric chloride (863 mg, 3.2 mmol) in triethylamine (0.51 ml) and dichloromethane (10 ml) at 0°C was added portionwise over 30 minutes S-methyl-N-phenyl-N'-[3-(6-{2,3,5,6-tetrahydroimidazo[2,1-*b*]thiazolyl})phenyl]isothiourea (1 g, 2.7 mmol). After a further 30 minutes the mixture was filtered and sodium (70 mg, 3 mmol) in dry methanol (30 ml) was added to the filtrate. This mixture was stirred overnight at room temperature, quenched with a drop of water and the solvent removed *in vacuo*. Purification of the crude product by preparative thin layer chromatography on alumina eluting with chloroform afforded the isourea (110 mg, 12%) as an oil.

(Found: *m/e* 352.1340 ($M^+$). Calculated for $C_{19}H_{20}N_4OS$: *m/e* 352.1358)

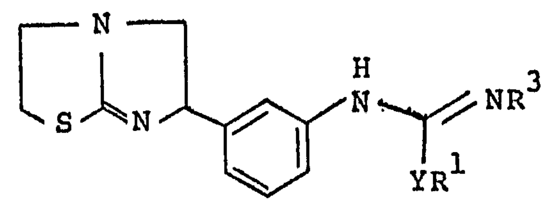

| Example | $R^3$ | $YR^1$ | Accurate Mass Measurement (m/e) | | m.p. (°C) |
|---|---|---|---|---|---|
| | | | Found | Calculated | |
| 50 | $C_6H_5$ | SPr | 320.1091 $[\underline{M}-HS^nPr]^+$ | 320.1095 ($C_{18}H_{16}N_4S$) | 52-5 |
| 51 | $C_6H_5$ | $S^iPr$ | 396.1411 $[M]^+$ | 396.1440 ($C_{21}H_{24}N_4S_2$) | |
| 52 | $C_6H_5$ | SBu | 410.1595 $[\underline{M}]^+$ | 410.1597 ($C_{22}H_{26}N_4S_2$) | 57-60 |
| 53 | $C_6H_5$ | $SCH_2C_6H_5$ | 444.1414 $[M]^+$ | 444.1442 ($C_{25}H_{24}N_4S_2$) | 44-7 |
| 54 | $C_6H_5$ | $OCH_3$ | 352.1340 $[M]^+$ | 352.1358 ($C_{19}H_{20}N_4OS$) | |
| 55 | 1-naphthyl | $SCH_3$ | 370.1269 $[\underline{M}-HSCH_3]^+$ | 370.1250 ($C_{22}H_{18}N_4S$) | 48-50 |
| 56 | 3,4-$C_6H_3(CH_3)_2$ | $SCH_3$ | 396.1443 $[M]^+$ | 396.1439 ($C_{21}H_{24}N_4S_2$) | 59-61 |
| 57 | 3-$C_6H_4CH_3$ | $SCH_3$ | 382.1277 $[\underline{M}]^+$ | 382.1286 ($C_{20}H_{22}N_4S_2$) | 63-5 |
| 58 | 3-$C_6H_4NO_2$ | $SCH_3$ | | | 74-6 |
| 59 | 3-$C_6H_4Br$ | $SCH_3$ | 398.0197 $[M-HSCH_3]^+$ | 398.0198 ($C_{18}H_{15}BrN_4S$) | 48-52 |
| 60 | 4-$C_6H_4O\underline{p}NO_2C_6H_4$ | $SCH_3$ | 457.1193 $[\underline{M}-HSCH_3]^+$ | 457.1207 ($C_{24}H_{19}N_5O_3S$) | |
| 61 | 2-$C_6H_4Cl$ | $SCH_3$ | 402.0754 $[\underline{M}]^+$ | 402.0739 ($C_{19}H_{19}ClN_4S_2$) | 70-5 |
| 62 | 4-$C_6H_4CO_2C_2H_5$ | $SCH_3$ | 440.1346 $[\underline{M}]^+$ | 440.1338 ($C_{22}H_{24}N_4O_2S_2$) | 41-4 |
| 63 | 4-$C_6H_4SO_2CH_3$ | $SCH_3$ | 446.0917 $[\underline{M}]^+$ | 446.0902 ($C_{20}H_{22}N_4O_2S_3$) | 55 |

| Example | R | $YR^1$ | Accurate Mass Measurement (m/e) | | m.p. (°C) |
|---|---|---|---|---|---|
| | | | Found | Calculated | |
| 64 | $4-C_6H_4SO_2N$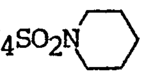 | $SCH_3$ | | | 80–5 |
| 65 | $4-C_6H_4SCH_3$ | $SCH_3$ | 414.1029 [M]$^+$ | 414.1006 ($C_{20}H_{22}N_4S_3$) | |
| 66 | $4-C_6H_4SO_2N(CH_3)_2$ | $SCH_3$ | | | 86–90 |
| 67 | $4-C_6H_4S$p$NO_2C_6H_4$ | $SCH_3$ | 473.1013 [M-$HSCH_3$]$^+$ | 473.0978 ($C_{24}H_{19}N_5O_2S_2$) | 60–6 |
| 68 | $4-C_6H_4CF_3$ | $SCH_3$ | 436.0980 [M]$^+$ | 436.1000 ($C_{20}H_{19}F_3N_4S_2$) | 58–62 |
| 69 | $4-C_6H_4CO.C_6H_5$ | $SCH_3$ | 424.1335 [M-$HSCH_3$]$^+$ | 424.1356 ($C_{25}H_{20}N_4OS$) | 155–8 |
| 70 | $4-C_6H_4SPr$ | $SCH_3$ | 442.1300 [M]$^+$ | 442.1316 ($C_{22}H_{26}N_4S_3$) | 46–8 |
| 71 | $4-C_6H_4SO.CH_3$ | $SCH_3$ | 382.0931 [M-$HSCH_3$]$^+$ | 382.0921 ($C_{19}H_{18}N_4OS_2$) | 75–80 |
| 72 | 3-C6H4CN | SCH3 | 345.1027 [M-HSCH3]$^+$ | 345.1048 (C19H15N5S) | 55–9 |
| 73 | $4-C_6H_4SO.Pr$ | $SCH_3$ | 410.1223 [M-$HSCH_3$]$^+$ | 410.1233 ($C_{21}H_{22}N_4OS_2$) | 70–3 |
| 74 | $4-C_6H_4Br$ | $SCH_3$ | 398.0234 [M-$HSCH_3$]$^+$ | 398.0199 ($C_{18}H_{15}BrN_4S$) | 58–60 |
| 75 | $CH_2C_6H_5$ | $SCH_3$ | 382.1273 [M]$^+$ | 382.1286 ($C_{20}H_{22}N_4S_2$) | |
| 76 | c-$C_6H_{11}$ | $SCH_3$ | 374.1603 [M]$^+$ | 374.1599 ($C_{19}H_{26}N_4S_2$) | |
| 77 | n-$C_6H_{13}$ | $SCH_3$ | 376.1761 [M]$^+$ | 376.1753 ($C_{19}H_{28}N_4S_2$) | |
| 78 | $CO.C_6H_5$ | $SCH_3$ | 396.1074 [M]$^+$ | 396.1078 ($C_{20}H_{20}N_4OS_2$) | 41–5 |

Example 79

N-Phenyl-N'-[3-(6-{5,6-dihydroimidazo[2,1-b]thiazolyl})-phenyl]thiourea

To a solution of 6-(m-aminophenyl)-5,6-dihydroimidazo [2,1-b]thiazole (4.3g, 19.8 mmol) in acetone (50 ml) was added phenylisothiocyanate (5.36 g, 39.6 mmol). The mixture was stirred overnight at room temperature, the precipitate filtered, washed with acetone and dried under vacuum to give a white solid (5.1 g).

The solid was suspended in chloroform (100 ml) and ammonia solution (25 ml, sg 0.88) added. The mixture was stirred at room temperature for 2 hours by which time the solid had all dissolved. The layers were separated and the aqueous phase extracted with chloroform. The combined chloroform extracts were dried (magnesium sulphate), evaporated, and the residue crystallised from chloroform to give N-phenyl-N'-[3-(6-{5,6-dihydroimidazo [2,1-b]-thiazolyl})phenyl]thiourea as a white solid which contained chloroform of crystallisation (nmr data), mp 102-4°C.

Microanalysis

Found: C: 51.76; H: 3.87; N: 12.98; S: 15.22. $C_{18}H_{16}N_4S_2$ + 0.68 $CHCl_3$ requires C: 51.75; H: 3.88; N: 12.92; S: 14.79.

Example 80

N-(4-Cyanophenyl)-N'-[3-(6-{5,6-dihydroimidazo[2,1-b]-thiazolyl})phenyl]thiourea was prepared by a method analogous to that of Example 79.
mp 119-121°C.

Example 81

S-Methyl-N-phenyl-N'-[3-(6-{5,6-dihydroimidazo[2,1-b]-thiazolyl})phenyl]isothiourea was prepared by a method analogous to that of Example 13.
Found: m/e 366.1000 ($M^+$). Calculated for $C_{19}H_{18}N_4S_2$: m/e 366.0971.

Example 82

S-Methyl-N-(4-cyanophenyl)-N'-[3-(6-{5,6-dihydroimidazo-[2,1-b]thiazolyl})phenyl]isothiourea was prepared by a method analogous to that of Example 13.

Example 83 to 91

The compounds of Examples 83 to 91 were prepared by a method analogous to that of Example 1 .

Examples 92 to 99

The compounds of Examples 92 to 99 were prepared by a method analogous to that of Example 13.

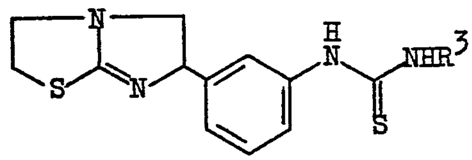

| Example | $R^3$ | ELEMENTAL ANALYSIS Found F Calculated C | C | H | N | S | mp (°C) |
|---|---|---|---|---|---|---|---|
| 83 | 4-$C_6H_4NHC_6H_5$ | | | | | | 143-8 |
| 84 | 3,5-$C_6H_3(NO_2)_2$ | | | | | | Decomp'n |
| 85 | 4-$C_6H_4CO_2H$ + 0.66 $H_2O$ | F<br>C | 55.82<br>55.60 | 4.59<br>4.71 | 13.11<br>13.65 | 15.20<br>15.60 | |
| 86 | 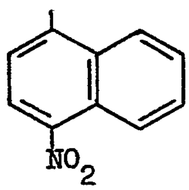 | F<br>C | 58.05<br>58.78 | 4.54<br>4.26 | 15.63<br>15.58 | 14.19<br>14.26 | 168-70 |
| 87 | 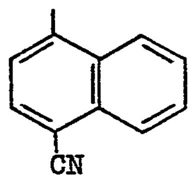 | F<br>C | 63.63<br>64.31 | 4.47<br>4.46 | 15.93<br>16.30 | 14.44<br>14.93 | 148-50 |
| 88 | 4-$C_6H_4SO_2$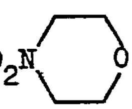 | F<br>C | 52.11<br>52.49 | 4.91<br>4.97 | 13.66<br>13.92 | 18.56<br>19.09 | 141-2 |
| 89 | 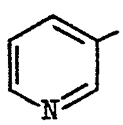 | F<br>C | 57.07<br>57.46 | 4.67<br>4.79 | 18.97<br>19.72 | 17.18<br>18.03 | 150-2 |
| 90 | 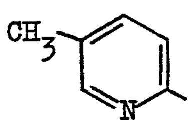 | F<br>C | 58.83<br>58.54 | 5.19<br>5.15 | 19.05<br>18.97 | 17.30<br>17.34 | 155-7 |
| 91 | 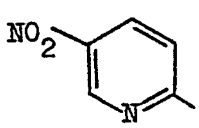 | | | | | | 190-200 |

0076622

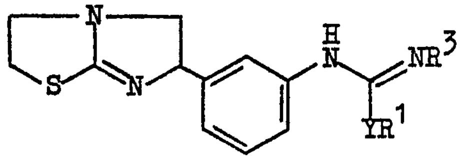

| Example | $R^3$ | $YR^1$ | Accurate Mass Measurement (m/e) Found | Accurate Mass Measurement (m/e) Calculated | mp (°C) |
|---|---|---|---|---|---|
| 92 | 4-$C_6H_4NH.C_6H_5$ | $SCH_3$ | | | 65-7 |
| 93 | 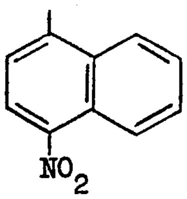 | $SCH_3$ | | | 100-5 |
| 94 | 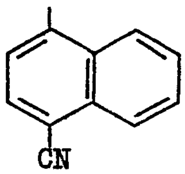 | $SCH_3$ | 395.1179 [M-$HSCH_3$]$^+$ | 395.1204 ($C_{23}H_{17}N_5S$) | 75-8 |
| 95 | 4-$C_6H_4SO_2$N 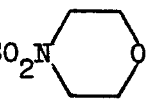 | $SCH_3$ | | | 100-2 |
| 96 | 2-$C_6H_4CN$ | $SCH_3$ | 392.1005 [M-H]$^+$ | 392.1001 ($C_{20}H_{18}N_5S_2$) | 78-80 |
| 97 | 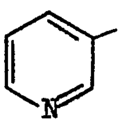 | $SCH_3$ | 321.1053 [M-$HSCH_3$]$^+$ | 321.1046 ($C_{17}H_{15}N_5S$) | 48-51 |
| 98 | 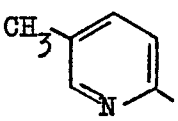 | $SCH_3$ | 383.1231 [M]$^+$ | 383.1237 ($C_{19}H_{21}N_5S_2$) | |
| 99 | 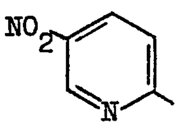 | $SCH_3$ | 367.0971 [M-$SCH_3$]$^+$ | 367.0974 ($C_{17}H_{15}N_6O_2S$) | |

Pharmacological Data

The oral activity of the following compounds, against experimental infections of Nematospiroides dubius in the mouse, was calculated from a comparison of the worm numbers of treated and control (untreated) animals.

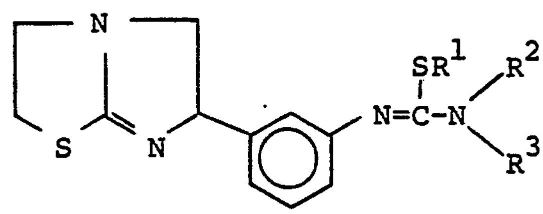

| Example No | $R^1$ | $R^2$ | $R^3$ | Dose mg/kg (p.o.) | Activity % |
|---|---|---|---|---|---|
| 13 | $CH_3$ | H | $C_6H_5$ | 200 | 99 |
| 14 | $CH_3$ | H | p-$C_6H_4F$ | 200 | 100 |
| 15 | $CH_3$ | H | m-$C_6H_4F$ | 200<br>100 | 100<br>100 |
| 16 | $CH_3$ | H | p-$C_6H_4NO_2$ | 200<br>100<br>50 | 100<br>100<br>95 |
| 22 | $C_2H_5$ | H | $C_6H_5$ | 200 | 92 |
| 17 | $CH_3$ | H | p-$C_6H_4Cl$ | 200 | 88 |
| 18 | $CH_3$ | H | 3,4-$C_6H_3Cl_2$ | 200 | 34 |
| 19 | $CH_3$ | H | p-$C_6H_4CH_3$ | 200 | 0 |
| 20 | $CH_3$ | H | m-$C_6H_4Cl$ | 200<br>100 | 100<br>87 |
| 56 | $CH_3$ | H | 3,4-$C_6H_3(CH_3)_2$ | 200 | 0 |
|  | $CH_3$ | H | p-$C_6H_4OCH_3$ | 200 | 59 |
| 57 | $CH_3$ | H | m-$C_6H_4CH_3$ | 200<br>100 | 100<br>40 |

| Example No | $R^1$ | $R^2$ | $R^3$ | Dose mg/kg (p.o.) | Activity % |
|---|---|---|---|---|---|
| 58 | $CH_3$ | H | m-$C_6H_4NO_2$ | 200<br>100 | 95<br>95 |
| 50 | $C_3H_7$ | H | $C_6H_5$ | 200 | 42 |
| 75 | $CH_3$ | H | $CH_2C_6H_5$ | 200 | 54 |
| 76 | $CH_3$ | H | Cyclohexyl | 200 | 15 |
| 25 | $CH_3$ | H | p-$C_6H_4COCH_3$ | 200<br>100 | 100<br>93 |
| 24 | $CH_3$ | H | p-$C_6H_4CN$ | 200<br>100 | 100<br>99 |

CLAIMS

1. A compound of formula (I):

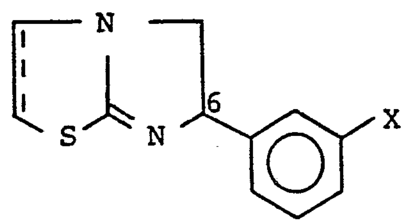

(I)

having the 6-S-absolute configuration, or, where appropriate, a salt thereof wherein X is selected from:

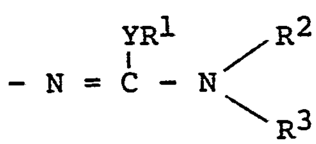

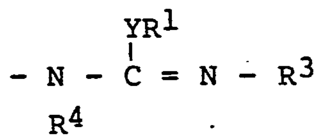

and

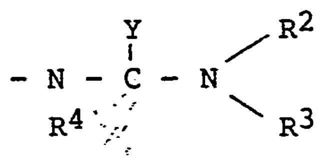

Y is oxygen, or sulphur

$R^1$ is alkyl or aralkyl

$R^2$, $R^3$ and $R^4$ are the same or different and each is selected from hydrogen, alkyl, aryl, substituted aryl, aralkyl,

substituted aralkyl, and, in addition $R^2$ and $R^3$ are also selected from heterocyclyl and substituted heterocyclyl,except that when Y is oxygen, $R^2$ and $R^4$ are hydrogen,

and the dotted line is an optionally present carbon-carbon bond.

2. A compound as claimed in claim 1 wherein $R^2$ and $R^4$ are hydrogen.

3. An isothiourea as claimed in claim 1 wherein $R^1$ is methyl.

4. A process for producing a compound of formula (I) as defined in claim 1, which process comprises either

a) reacting a compound of formula (I) or the 6-R-isomer thereof wherein X is a group

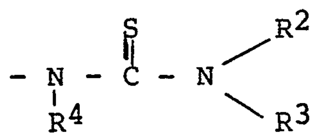

or a salt thereof with a compound of formula (II):

$$Z - R^1 \qquad \text{(II)}$$

wherein Z is a leaving group and, if necessary, thereafter racemising the product, to produce a compound of formula (I) wherein X is

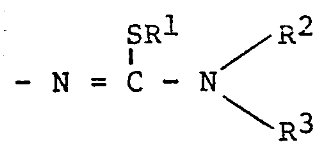

or

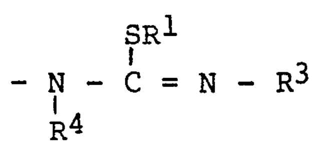

or b) reacting a compound of formula (III):

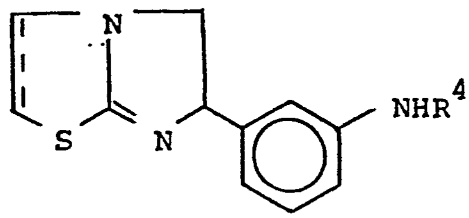

(III)

with a compound of formula (IV):

$$Y = C = N - R^3 \qquad (IV)$$

and, if necessary, thereafter racemising the product.

or c) reacting the compound of formula (V):

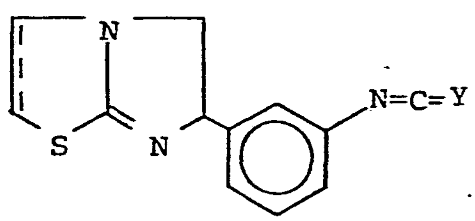

(V)

with a compound of formula (VI):

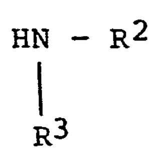

(VI)

and, if necessary, thereafter racemising the product.

or d) converting a compound of formula (I), or the 6-R-isomer thereof wherein X is

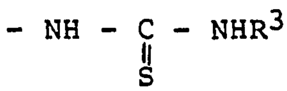

or

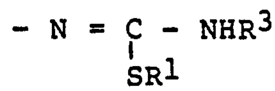

to a compound wherein X is

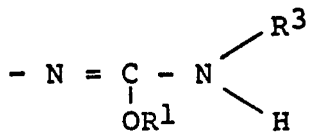

and, if necessary, thereafter racemising the product.

5. A pharmaceutical or veterinary composition comprising a compound of formula (I) as defined in claim 1 and a pharmaceutically acceptable carrier therefor.

6. A composition as claimed in claim 5 wherein the composition is a solution or suspension for oral administration.

7. A composition as claimed in claim 5 wherein the carrier is a sustained release device.

8. A process for producing a pharmaceutical or veterinary composition as claimed in claim 5 which process comprises bringing into association the compound of formula (I) and the carrier therefor.

9. A compound of formula (I) as defined in claim 1 for use in treating the human or animal body.

European Patent Office

# EUROPEAN SEARCH REPORT

0076622

Application number

EP 82 30 5126.3

## DOCUMENTS CONSIDERED TO BE RELEVANT

| Category | Citation of document with indication, where appropriate, of relevant passages | Relevant to claim |
|---|---|---|
| | GB - A - 1 365 515 (AMERICAN CYANAMID CO.) | |
| X | * claims 1, 5 to 9 ; page 15, table, example 78 * | 1,2 |
| A | * claims 15, 16 ; page 3, lines 12 to 16 * | 5,6 |
| | -- | |
| | US - A - 3 899 583 (L.D. SPICER et al.) | |
| X | * claims 1, 2 ; column 1, line 32 to column 2, line 39 ; columns 37, 38, table II, examples 105, 108 * | 1,2 |
| A | * claims 1, 2 * | 5,9 |
| | -- | |
| P,A | EP - A1 - 0 041 322 (BEECHAM GROUP LTD.) * claims 1, 5, 8, 10, 11 * | 1,4,5, 9 |
| | -- | |
| A | US - A - 4 208 419 (C.J. PAGET et al.) * columns 4, 5 ; reaction scheme I, example 1, reaction scheme II * | 4 |
| | -- | |
| D,A | EP - A1 - 0 010 987 (BEECHAM GROUP LTD.) * abstract * | 7 |
| | ---- | |

**CLASSIFICATION OF THE APPLICATION (Int. Cl. 3)**

C 07 D 513/04
A 61 K 31/425
//(C 07 D 513/04, 277/00, 235/00)

**TECHNICAL FIELDS SEARCHED (Int.Cl. 3)**

A 61 K 31/425
C 07 D 513/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 30-12-1982 | HASS |

EPO Form 1503.1 06.78